Europäisches Patentamt

European Patent Office

Office européen des brevets

(11) Veröffentlichungsnummer: **0 210 332**
**B1**

(12) EUROPÄISCHE PATENTSCHRIFT

(45) Veröffentlichungstag der Patentschrift:
10.01.90

(51) Int. Cl.⁴: **C12N 9/64**, C12N 9/72

(21) Anmeldenummer: 86104477.4

(22) Anmeldetag: 02.04.86

(54) Verfahren zur Reinigung von Plasminogenaktivatoren (PA).

(30) Priorität: 11.04.85 DE 3512910

(43) Veröffentlichungstag der Anmeldung:
04.02.87 Patentblatt 87/6

(45) Bekanntmachung des Hinweises auf die Patenterteilung:
10.01.90 Patentblatt 90/2

(84) Benannte Vertragsstaaten:
AT BE CH DE FR GB IT LI LU NL SE

(56) Entgegenhaltungen:
CH-A- 647 548
FR-A- 2 362 155

CHEMICAL ABSTRACTS, Band 90, Nr. 3, 15.
Januar 1979, Seite 245, Nr. 18309a, Columbus, Ohio, US;
& JP - A - 78 96 384 (TEIJIN LTD.) 23-08-1978
THE JOURNAL OF BIOCHEMISTRY, Band 92, Nr. 4, 1982,
Seiten 1129-1140, Tokyo, JP; Y. HISHIKAWA-ITOH et al.:
"Streptokinase-dependent potentiating factor
(SK-potentiator) for plasminogen activation from
human plasma: its identification as a fibrinogen
degradation product" 000

(73) Patentinhaber: BEHRINGWERKE Aktiengeseilschaft,
Postfach 1140, D-3550 Marburg 1(DE)

(72) Erfinder: Pâques, Eric Paul, Dr., Schmiedeacker 18,
D-3550 Marburg(DE)

(74) Vertreter: Klein, Otto, Dr. et al, Hoechst AG Zentrale
Patentabteilung Postfach 80 03 20, D-6230 Frankfurt am
Main 80(DE)

## Beschreibung

Die Erfindung betrifft ein Verfahren zur Reinigung von Plasminogenaktivatoren (PA). Unter dem Begriff Plasminogenaktivator sollen Proteine mit Gewebe-Plasminogenaktivator - (t-PA) Aktivitäten verstanden werden sowie auch natürlich vorkommende, synthetisch oder gentechnisch hergestellte Derivate derselben.

Plasminogen wird durch Plasminogenaktivatoren in Plasmin umgewandelt. Zu den Katalysatoren dieser Reaktion zählen Urokinase und t-PA. Die therapeutische Anwendung dieser Aktivatoren als Fibrinolytika ist bekannt. Aufgrund seiner hohen Affinität zu Fibrin ist t-PA für die Lyse-Therapie von besonderer Bedeutung.

Es sind bereits Methoden zur Isolierung und Reinigung von PA aus Zellkulturüberständen und Urin beschrieben worden (DE-OS 28 15 853, EP 00 41 766). Diese Verfahren sind umständlich und daher für eine großtechnische Anwendung nicht geeignet.

In EP 0 041 766 wird ein Verfahren zur Isolierung von t-PA beschrieben. Hierfür werden Zink-Chelat-Sepharose[R], Concanavalin-A-Agarose[R] und Sephadex[R] G-150 (Superfine) verwendet. Jeder dieser Reinigungsschritte ist mit großen Nachteilen verbunden: Zink und Concanavalin A können das Produkt kontaminieren und Sephadex[R] G-150 (Superfine) ist aufgrund seiner Kapazität und seiner Durchflußeigenschaften für ein großtechnisches Verfahren nicht anwendbar. In EP 0 023 869 wird die Reinigung von t-PA mit einem Träger, an dem lösliche Fragmente von Fibrin durch kovalente Bindung fixiert sind, beschrieben. Dieses Verfahren eignet sich ebenfalls nicht für großtechnische Isolierungsverfahren.

Der vorliegenden Erfindung liegt die Aufgabe zugrunde, ein einfaches Reinigungsverfahren für t-PA zu entwickeln.

Es wurde überraschenderweise gefunden, daß die oben bezeichneten PA eine hohe Affinität zu Polyschwefelsäureestern von Sacchariden oder sulfatierten Zuckern aufweisen und eine Reinigung dieser t-PA über einen solchen Schwefelsäureester, wenn dieser an einen Träger gebunden ist, möglich ist.

Gegenstand der Erfindung ist deshalb ein Verfahren für die Reinigung eines Gewebe-Plasminogenaktivators, dadurch gekennzeichnet, daß eine einen dieser Aktivatoren enthaltende Lösung mit einem trägergebundenen Polyschwefelsäureester eines Saccharids oder sulfatierten Zucker ("Affinitätsmaterial") in Kontakt gebracht, die Flüssigkeit abgetrennt und der von diesem Material gebundene Aktivator eluiert wird.

Plasminogenaktivatoren sind Proteine oder Gewebeplasminogenaktivator- Aktivität sowie deren natürlich vorkommenden, synthetisch oder gentechnisch hergestellten Derivate.

Vorzugsweise werden vor der Elution des Aktivators durch Waschen vom Affinitätsmaterial gebundene Verunreinigungen entfernt.

Bevorzugt ist weiterhin eine Verfahrensweise, bei der das beladene Affinitätsmaterial mit einem Puffer, enthaltend Natriumsulfat, Ammoniumsulfat, NaCl, LiCl oder Citrat von Verunreinigungen befreit und der Aktivator mit einer Pufferlösung, enthaltend Kaliumnitrat, Ammoniumchlorid, Bariumchlorid, Kaliumbromid, Calciumchlorid, Magnesiumchlorid, Kaliumthiocyanat, Harnstoff oder einer Mischung dieser Stoffe eluiert wird.

Als Trägermaterialien für eine kovalente Kupplung von Polyschwefelsäureestern eines Saccharids oder sulfatierten Zuckern können beispielsweise unlösliche Agarose-, Dextran-, Acrylamid - oder Polyethylenglycolglycidylmethacrylat-Polymeren oder deren Kombination angewendet werden. Bevorzugt werden Dextran- oder Agarose-Matrices.

Die Kupplung von Polyschwefelsäureestern von Sacchariden oder sulfatierten Zuckern erfolgt nach bekannten Methoden wie zum Beispiel durch Bindung an Bromcyan-voraktiviertes Trägermaterial, beziehungsweise an aminofunktionalisiertes Harz mittels Carbodiimid-Kondensation, bevorzugt aber durch Kupplung an Lysin-funktionalisiertes Trägermaterial mittels Carbodiimid-Kondensation.

Als Polyschwefelsäureester eines Saccharids oder sulfatierte Zucker können Dextran-Sulfat, Heparan-Sulfat, Chondroitin-Sulfat, Keratan-Sulfat, Dermatan-Sulfat, Pentosan-Sulfat, Arteparon[R], vorzugsweise Heparin, angewendet werden.

Eine weitere vorteilhafte Verfahrensweise besteht darin, daß die Plasminogenaktivator enthaltende Lösung mit einem trägergebundenen Polyschwefelsäureester eines Saccharids oder sulfatierten Zucker, vorzugsweise Heparin-Lysin-Sepharose, zusammengebracht wird, das beladene Affinitätsmaterial mit einem Puffer von pH 3 bis 9, der gegebenenfalls NaCl enthält, gewaschen wird, die am Harz gebundenen Verunreinigungen mit einem Puffer von pH 3 bis 9 enthaltend Natriumsulfat, Ammoniumsulfat, NaCl, LiCl oder Citrat, vorzugsweise mit einer 0,1 bis 2 mol/l Citrat-Lösung von pH 3 bis 9, vorzugsweise mit 0,5 mol/l Citrat pH 3,5 bis 6, eluiert werden und der Plasminogenaktivator mit einem Puffer von pH 3 bis 9 enthaltend $KNO_3$, KSCN, $NH_4Cl$, $CaCl_2$, $MgCl_2$, KBr, $BaCl_2$ oder Harnstoff, vorzugsweise 1 bis 2 mol/l KSCN, vorzugsweise pH 5-8, enthaltend gegebenenfalls Detergenzien, vorzugsweise 0,1 bis 1 g/l Tween[R] 80 (Polyoxyethylensorbitan-monooleat) eluiert wird.

In einer besonders bevorzugten Ausführungsform kann man so verfahren, daß man die PA enthaltende Lösung, beispielsweise Melanom-Zellkulturüberstände oder Urin, gegebenenfalls entsalzt, mit Heparin-, Dextran-Sulfat- oder Pentosan-Sulfat-Sepharose[R], vorzugsweise Heparin-Sepharose[R], bevorzugt Heparin-Lysin-Sepharose[R], vorzugsweise in einem Verhältnis von 10 l Zellkulturüberstand zu 100 g Affinitätsmaterial versetzt, das Harz mit einer 0,1 bis 2 mol/l Citrat-Lösung, pH 3-9, vorzugsweise mit 0,5 mol/l Citrat, pH 3,5-6 von Verunreinigungen befreit, und Plasminogenaktivator mit einem Puffer enthaltend 1-2 mol/l KSCN, pH 5-8, gegebenenfalls enthaltend 0,1 - 1 g/l Tween[R] 80 eluiert.

In einer weiteren, besonders bevorzugten Ausführungsform, kann man auch so verfahren, daß man PA mit einer Pufferlösung, enthaltend 1 bis 2

mol/l $CaCl_2$ mit einem pH-Wert von 4 bis 9, vorzugsweise mit einer 2 mol/l $CaCl_2$-Lösung von pH 5 bis 8, enthaltend gegebenenfalls 0,1 bis 1 g/l Tween[R] 80, eluiert.

Das Verfahren zeichnet sich dadurch aus, daß PA mit einem Reinigungsschritt in einem Reinheitsgrad bzw. einer spezifischen Aktivität erhalten werden kann, die nach üblichen Verfahren erst nach mehreren Reinigungsschritten erhalten sind.

Die Erfindung wird durch die nachstehenden Beispiele erläutert.

Beispiel 1

10 l Zellkulturüberstand von t-PA produzierenden Melanomzellen wurden mit 100 g einer Heparin-Sepharose[R] bei Raumtemperatur unter Rühren versetzt. Das Affinitätsmaterial wurde nach Abtrennen der Proteinflüssigkeit mit 0,1 mol/l Tris-HCl, 0,1 mol/l NaCl, pH 7,5, enthaltend 0,1 % Tween[R] 80, gewaschen und anschließend mit 0,5 mol/l Citrat pH 5,0 von Verunreinigungen befreit. Der PA wurde mit einem Puffer, enthaltend 0,1 mol/l Tris-HCl, 2 mol/l KSCN und 0,01 % Tween[R] 80 pH 7,5 eluiert. Das Eluat wurde dialysiert, konzentriert und auf t-PA-Aktivität getestet. Von der in der Ausgangslösung vorhandenen t-PA-Aktivität wurden 99 % an Heparin-Sepharose gebunden. Nach Elution wurden etwa 90 % der t-PA-Aktivität wiedergefunden.

Eine Polyacrylamidgelelektrophorese zeigte zwei Proteinbanden unterschiedlichen Molekulargewichts. Eine Bande konnte immunologisch dem t-PA zugeordnet werden.

Beispiel 2

10 l Zellkulturüberstand wurden wie in Beispiel 1 angegeben behandelt, anschließend mit einer 2 mol/l $CaCl_2$-Lösung enthaltend 0,1 mol/l Tris, pH 8,0, und 0,1 % Tween[R] 80, eluiert. Die Ergebnisse waren vergleichbar den in Beispiel 1 angegebenen.

Beispiel 3

10 l Urin wurden durch Dialyse entsalzt, anschließend mit 100 g einer Heparin-Sepharose[R] bei Raumtemperatur unter Rühren versetzt. Das Affinitätsmaterial wurde nach Abtrennen der Proteinflüssigkeit mit 0,1 mol/l Tris-HCl pH 7,5 gewaschen, danach mit einer Pufferlösung enthaltend 2 mol/l KSCN, 0,1 mol/l Tris-HCl, pH 7,5 eluiert. 80% der Ausgangsaktivität wurde an Heparin-Sepharose gebunden. Nach Elution konnte etwa 80 % der Urokinase-Aktivität wiedergefunden werden.

Beispiel 4

10 l Zellkulturüberstand von t-PA produzierenden Melanomzellen wurden mit 500 g Pentosan-Sulfat-Sepharose versetzt und wie in Beispiel 3 aufgearbeitet. Der am Harz gebundene Aktivitätsanteil war etwa 80 %.

**Patentansprüche**

1. Verfahren für die Reinigung eines Gewebe-Plasminogenaktivators (t-PA), dadurch gekennzeichnet, daß ein Gewebe-Plasminogenaktivator oder eines seiner Derivate enthaltende Lösung mit einem trägergebundenen Polyschwefelsäureester eines Saccharids oder sulfatierten Zucker ("Affinitätsmaterial") in Kontakt gebracht, die Flüssigkeit abgetrennt, das Affinitätsmaterial von Verunreinigungen befreit und von diesem Material gebundener t-PA oder gebundenes t-PA-Derivat eluiert wird.

2. Verfahren nach Anspruch 1, dadurch gekennzeichnet, daß der Polyschwefelsäureester eines Saccharids oder sulfatierten Zucker über Lysin an einem Träger gebunden ist.

3. Verfahren nach Anspruch 1, dadurch gekennzeichnet, daß der Polyschwefelsäureester eines Saccharids Heparin ist.

4. Verfahren nach Anspruch 1, dadurch gekennzeichnet, daß der Träger Agarose ist.

5. Verfahren nach Anspruch 1, dadurch gekennzeichnet, daß vor der Elution des Gewebe-Plasminogenaktivators die gebundenen Verunreinigungen durch Waschen des Affinitätsmaterials mit einer Pufferlösung, enthaltend NaCL, Natriumsulfat, Ammoniumsulfat, LiCL oder Alkalicitrat und gegebenenfalls 0,1 bis 1 g/l Polyoxyethylensorbitan-monooleat, entfernt werden.

6. Verfahren nach Anspruch 1, dadurch gekennzeichnet, daß vor der Elution des Gewebe-Plasminogenaktivators die gebundenen Verunreinigungen durch Waschen des Affinitätsmaterials mit einer 0,1 bis 2 mol/l Citrat-Lösung, pH 3-9, enthaltend gegebenenfalls 0,1 bis 1 g/l Polyoxyethylensorbitan-monooleat, entfernt werden.

7. Verfahren nach Anspruch 1, dadurch gekennzeichnet, daß vor der Elution des Gewebe-Plasminogenaktivators die gebundenen Verunreinigungen durch Waschen des Affinitätsmaterials mit einer 0.4 bis 0.6 mol/l Citrat-Lösung, pH 3,5 - 6, enthaltend gegebenenfalls 0,1 bis 1 g/l Polyoxyethylensorbitan-monooleat, entfernt werden.

8. Verfahren nach Anspruch 1, dadurch gekennzeichnet, daß das beladene Affinitätsmaterial mit einem Puffer von Verunreinigungen befreit und der Gewebe-Plasminogenaktivator mit einer Lösung von Kaliumnitrat, Ammoniumchlorid, Calciumchlorid, Magnesiumchlorid, Kaliumbromid, Bariumchlorid, Harnstoff oder Kaliumthiocyanat oder einer Mischung dieser Substanzen, enthaltend gegebenenfalls 0,1 bis 1 g/l Polyoxyethylensorbitan-monooleat, eluiert wird.

9. Verfahren nach Anspruch 1, dadurch gekennzeichnet, daß das beladene Affinitätsmaterial mit einem Puffer von Verunreinigungen befreit und der Gewebe-Plasminogenaktivator mit einer Puffer-Lösung enthaltend 0,5 bis 2 mol/l KSCN, pH 4 bis 9, und gegebenenfalls 0,1 bis 1 g/l Polyoxyethylensorbitan-monooleat, eluiert wird.

10. Verfahren nach Anspruch 1, dadurch gekennzeichnet, daß das beladene Affinitätsmaterial mit einem Puffer von pH 4 bis 9, der gegebenenfalls NaCl enthält, gewaschen wird, die gebundenen Verunreinigungen durch Waschen des Affinitätsmaterials mit einer 0,4 bis 0,6 mol/l Alkalicitrat-Lösung, pH 3,5-6, enthaltend gegebenenfalls 0,1 bis 1 g/l Polyoxyethylensorbitan-monooleat, entfernt werden und der Plasminogenaktivator mit einer Pufferlösung, enthaltend 0,5 bis 2 mol/l KSCN, pH 4-9, enthaltend gegebenenfalls 0,1 bis 1 g/l Polyoxyethylensorbitan-monooleat, eluiert wird.

## Claims

1. A process for the purification of a tissue plasminogen activator (t-PA), which comprises bringing a solution containing tissue plasminogen activator or one of its derivatives into contact with a carrier-bound polysulfate of a saccharide or sulfated sugar (affinity material), removal of the liquid, freeing the affinity material from impurities, and elutin t-PA, or t-PA derivative, bound by this material.

2. The process as claimed in claim 1, wherein the polysulfate of a saccharide or sulfated sugar is bound via lysine to a carrier.

3. The process as claimed in claim 1, wherein the polysulfate of a saccharide is heparin.

4. The process as claimed in claim 1, wherein the carrier is agarose.

5. The process as claimed in claim 1, wherein before elution of the tissue plasminogen activator the bound impurities are removed by washing the affinity material with a buffer solution containing NaCl, sodium sulfate, ammonium sulfate, LiCl or alkali metal citrate and, where appropriate, 0.1 to 1 g/l polyoxyethylene sorbitan monooleate.

6. The process as claimed in claim 1, wherein before the elution of the tissue plasminogen activator the bound impurities are removed by washing the affinity material with a 0.1 to 2 mol/l citrate solution, pH 3–9, containing, where appropriate, 0.1 to 1 g/l polyoxyethylene sorbitan monooleate.

7. The process as claimed in claim 1, wherein before elution of the tissue plasminogen activator the bound impurities are removed by washing the affinity material with a 0.4 to 0.6 mol/l citrate solution, pH 3.5–6, containing, where appropriate, 0.1 to 1 g/l polyoxyethylene sorbitan monooleate.

8. The process as claimed in claim 1, wherein the loaded affinity material is freed from impurities with a buffer, and the tissue plasminogen activator is eluted with a solution of potassium nitrate, ammonium chloride, calcium chloride, magnesium chloride, potassium bromide, barium chloride, urea or potassium thiocyanate or a mixture of these substances, containing, where appropriate, 0.1 to 1 g/l polyoxyethylene sorbitan monooleate.

9. The process as claimed in claim 1, wherein the loaded affinity material is freed from impurities with a buffer, and the tissue plasminogen activator is eluted with a buffer solution containing 0.5 to 2 mol/l KSCN, pH 4 to 9 and, where appropriate, 0.1 to 1 g/l polyoxyethylene sorbitan monooleate.

10. The process as claimed in claim 1, wherein the loaded affinity material is washed with a buffer of pH 4 to 9, which, where appropriate, contains NaCl, the bound impurities are removed by washing the affinity material with a 0.4 to 0.6 mol/l alkali metal citrate solution, pH 3.5–6, containing, where appropriate, 0.1 to 1g/l polyoxyethylene sorbitan monooleate, and the plasminogen activator is eluted with a buffer solution containing 0.5 to 2 mol/l KSCN, pH 4–9, containing, where appropriate, 0.1 to 1 g/l polyoxyethylene sorbitan monooleate.

## Revendications

1. Procédé pour la purification d'un activateur tissulaire du plasminogène (t-PA), caractérisé en ce que l'on met en contact une solution contenant de l'activateur tissulaire du plasmonogène ou un de ses dérivés, avec un polysulfate d'un saccharide ou un sucre sulfaté ("matériau d'affinité"), fixé sur un support on sépare le liquide, on élimine les impuretés hors du matériau d'affinité et of élue hors de ce matériau le t-PA fixé ou le dérivé de t-PA fixé.

2. Procédé selon la revendication 1, caractérisé en ce que le polysulfate d'un saccharide ou le sucre sulfaté est fixé sur un support par l'intermédiaire de lysine.

3. Procédé selon la revendication 1, caractérisé en ce que le polysulfate d'un saccharide est l'héparine.

4. Procédé selon la revendication 1, caractérisé en ce que le support est l'agarose.

5. Procédé selon la revendication 1, caractérisé en ce que, avant l'élution de l'activateur tissulaire du plasminogène, on élimine les impuretés fixées, par lavage du matériau d'affinité avec une solution tampon contenant du NaCl, du sulfate de sodium, du sulfate d'ammonium, du LiCl ou un citrate alcalin, et éventuellement de 0,1 à 1 g/l de monooléate de polyoxyéthylènesorbitanne.

6. Procédé selon la revendication 1, caractérisé en ce que, avant l'élution de l'activateur tissulaire du plasminogène, on élimine les impuretés fixées, par lavage du matériau d'affinité avec une solution de citrate à 0,1–2 mole/l, pH 3–9, contenant éventuellement de 0,1 à 1 g/l de monooléate de polyoxyéthylènesorbitanne.

7. Procédé selon la revendication 1, caractérisé en ce que, avant l'élution de l'activateur tissulaire du plasminogène, on élimine les impuretés fixées, par lavage du matériau d'affinité avec une solution de citrate à 0,4–0,6 mole/l, pH 3,5–6, contenant éventuellement de 0,1 à 1 g/l de monooléate de polyoxyéthylènesorbitanne.

8. Procédé selon la revendication 1, caractérisé en ce qu'on élimine à l'aide d'un tampon les impuretés hors du matériau d'affinité chargé, et on élue l'activateur tissulaire du plasminogène à l'aide d'une solution de nitrate de potassium, de chlorure d'ammonium, de chlorure de calcium, de chlorure de magnésium, de bromure de potassium, de chlorure de baryum, d'urée ou de thiocyanate de potassium, ou à l'aide d'un mélange de ces substances, contenant éventuellement de 0,1 à 1 g/l de monooléate de polyoxyéthylènesorbitanne.

9. Procédé selon la revendication 1, caractérisé en ce qu'on élimine à l'aide d'un tampon les impuretés hors du matériau d'affinité chargé, et on élue l'activateur tissulaire du plasminogène à l'aide d'une solution tampon contenant de 0,5 à 2 moles/l de KSCN,

pH 4 à 9, et éventuellement 0,1 à 1 g/l de monooléate de polyoxyéthylènesorbitanne.

10. Procédé selon la revendication 1, caractérisé en ce qu'on lave le matériau d'affinité chargé, à l'aide d'un tampon à pH 4–9 qui contient éventuellement du NaCl, on élimine les impuretés fixées, par lavage du matériau d'affinité avec une solution de 0,4 à 0,6 mole/l d'un citrate alcalin, pH 3,5–6, contenant éventuellement de 0,1 à 1 g/l de monooléate de polyoxyéthylènesorbitanne, et on élue l'activateur du plasminogène à l'aide d'une solution tampon contenant de 0,5 à 2 moles/l de KSCN; pH 4–9, contenant éventuellement de 0,1 à 1 g/l de monooléate de polyoxyéthylènesorbitanne.